**Europäisches Patentamt**

**European Patent Office**

**Office européen des brevets**

(19)

(11) Veröffentlichungsnummer: **0 297 294 B1**

(12) **EUROPÄISCHE PATENTSCHRIFT**

(45) Veröffentlichungstag der Patentschrift: **18.03.92**

(51) Int. Cl.⁵: **A61K 37/54**

(21) Anmeldenummer: **88108656.5**

(22) Anmeldetag: **31.05.88**

(54) **Verfahren zur Herstellung einer Lösung hoher spezifischer Volumenaktivität von einem Protein mit Gewebe-Plasminogenaktivator (t-PA)-Aktivität, Lösung, enthaltend Protein mit t-PA-Aktivität und Verwendung der Lösung in der Human- und Veterinärmedizin.**

(30) Priorität: **05.06.87 DE 3718889**

(43) Veröffentlichungstag der Anmeldung:
**04.01.89 Patentblatt 89/01**

(45) Bekanntmachung des Hinweises auf die
Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 156 169**
**EP-A- 0 217 379**
**EP-A- 0 218 112**

(73) Patentinhaber: **BEHRINGWERKE Aktiengesellschaft**
**Postfach 1140**
**W-3550 Marburg 1(DE)**

(72) Erfinder: **Pâques, Eric Paul, Dr.**
**Schmiedeacker 18**
**W-3550 Marburg(DE)**
Erfinder: **Stöhr, Hans-Arnold**
**Schulstrasse 66**
**W-3552 Wetter(DE)**

(74) Vertreter: **Tergau, Ulrich, Dr. et al**
**HOECHST AKTIENGESELLSCHAFT Zentrale**
**Patentabteilung Postfach 80 03 20**
**W-6230 Frankfurt am Main 80(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung einer Lösung hoher spezifischer Volumenaktivität von einem Protein mit Plasminogenaktivator-Aktivität, eine gemäß dem beanspruchten Verfahren hergestellte Lösung und die Verwendung dieser Lösung in der Human- und Veterinärmedizin.

Der Organismus verfügt über zwei im Gleichgewicht stehende Systeme, um sich sowohl vor Blutverlust als auch vor Thrombosen zu schützen: Das Gerinnungssystem und das fibrinolytische System. Das Zusammenspiel zwischen beiden gewährleistet, daß zunächst zur Blutstillung unlösliche Fibrinpolymere entstehen, die während der Wundheilung durch den lytischen Vorgang der Fibrinolyse wieder abgebaut werden.

Thrombin und Plasmin stellen die Schlüsselenzyme beider Systeme dar. Unter physiologischen Bedingungen steht das dynamische Gleichgewicht zwischen dem Gerinnungs- und Fibrinolysesystem unter Kontrolle der thromboplastischen Aktivität von Thrombin und der thrombolytischen Aktivität von Plasmin. Zum Gleichgewicht tragen die jeweiligen Inhibitoren bei. Ein Übergewicht eines der beiden Systeme kann fatale Folgen haben, nämlich sowohl Blutungen und Thrombosen als auch Gefäßschäden.

Das thrombolytisch wirksame Plasmin ist eine relativ unspezifische, trypsinähnliche Serinprotease. Es wird in Form eines inaktiven Vorläufers, des Plasminogens, synthetisiert. Plasminogen zirkuliert als inaktiver Vorläufer im Blut und wird erst auf bestimmte Reize hin aktiviert. Die Umwandlung von Plasminogen in Plasmin wird durch Plasminogenaktivatoren katalysiert.

Die Aktivierung des Plasminogens kann durch vier verschiedene Plasminogenaktivator-Systeme geschehen:

1. Ein Faktor XII abhängiges System,
2. einen aus Streptokokken isolierten Plasminogenaktivator, die Streptokinase,
3. Gewebe-Plasminogenaktivator (t-PA) und
4. urinären Plasminogenaktivator (u-PA bzw. Urokinase).

Der Aktivierung über das Faktor XII-abhängige System kommt nur eine untergeordnete physiologische Bedeutung zu. Der bakterielle Plasminogenaktivator Streptokinase ist zwar nach wie vor von großer therapeutischer Bedeutung, hat jedoch, ebenso wie der urinäre Plasminogenaktivator Urokinase, den Nachteil, daß er nach Applikation bzw. Freisetzung im gesamten Gefäßsystem wirksam ist, d.h., nicht nur am Zielort zu einer Aktivierung des Plasminogens führt. Der Gewebe-Plasminogenaktivator schließlich ist ein in vielen Geweben und Gewebeflüssigkeiten vorhandenes Protein, das erst nach Bindung an Fibrin seine volle fibrinolytische Aktivität entfaltet. Die Plasminogenaktivierung durch t-PA ist somit eine spezifisch nur am Zielort ablaufende Reaktion, die nicht die Gefahr einer gleichzeitigen unspezifischen Proteolyse anderer Plasmaproteine mit sich bringt.

Eine Unterfunktion des fibrinolytischen Systems, gleich welcher Genese, kann zu Gefäßverschlüssen durch Thrombusbildung führen. Die Folgen solcher Thromben sind zum Beispiel Herzinfarkte, Lungenembolien oder auch Schlaganfälle. In der Prophylaxe und Therapie vieler auf Minderfunktion des fibrinolytischen Systems beruhender Erkrankungen spielt die Applikation von Plasminogenaktivatoren eine bedeutende Rolle. Idealerweise sollte der applizierte Plasminogenaktivator eine nebenwirkungsfreie, fibrinspezifische Lysetherapie erlauben. Diese Anforderung kann nur von t-PA erfüllt werden, da t-PA-Aktivierung, wie bereits erwähnt, von der Bindung an Fibrin abhängt. t-PA kann aus animalen Zellen in therapeutisch ausreichenden Mengen produziert werden. t-PA hat jedoch, genau wie Urokinase, im menschlichen Körper eine Halbwertzeit von nur 3 bis 8 Minuten. Daraus folgt, daß eine stete und kontinuierliche t-PA-Zufuhr ermöglicht werden muß, z.B. in Form einer intravaskulären Infusion. Gleichzeitig muß das Volumen der zugeführten Flüssigkeitsmenge möglichst gering gehalten werden, um Patienten mit Herz- oder Niereninsuffizienz nicht zusätzlich zu belasten. Für die Formulierung von t-PA-haltigen, physiologisch verträglichen parenteralen Lösungen muß daher eine möglichst hohe spezifische Volumenaktivität gefordert werden.

Unter den in EP-A 156 169 beschriebenen Bedingungen können durch Zusatz von Lysin oder Ornithin t-PA-Konzentrationen von 3000 bis 50 000 U/ml erreicht werden. Bei der erforderlichen therapeutischen Dosierung ergäbe sich daraus eine extreme Flüssigkeitszufuhr. Der Zusatz von Lysin oder Ornithin, wie er in der europäischen Patentanmeldung EP 156 169 beschrieben ist, kann nicht zu therapeutisch sinnvoll anwendbaren t-PA-haltigen Lösungen führen.

In der DE-OS 36 17 753 werden t-PA-Lösungen beschrieben, in denen durch eine Erniedrigung des pH-Wertes auf 2 bis 5 t-PA-Konzentrationen von bis zu 5000 000 U/ml erreicht werden. Dadurch wird zwar eine therapeutisch vertretbare t-PA-Konzentration in der Lösung erzielt, gleichzeitig jedoch die Infusion einer Lösung mit krass unphysiologischem pH-Wert in Kauf genommen. Die Applikation solcher Lösungen führt unweigerlich zu Hautirritationen und Gefäßschäden in der Nähe der Infusionsstelle.

EP-A-218 112 beschreibt in Beispiel 10 eine wäßrige Lösung von Gewebe-Plasminogenaktivator, die L-Arginin und L-Histidin enthält.

EP-A-156 169 offenbart in Beispiel 10 ein Verfahren zur Herstellung einer Gewebe-Plasminogenaktivator-Lösung unter Verwendung von Glycin und Ornithin im Elutions-Lösungsmittel.

Aufgabe der vorliegenden Erfindung war es daher, ein Verfahren zur Verfügung zu stellen, das die Herstellung hochkonzentrierter parenteral applizierbarer, physioloigsch verträglicher Lösungen eines Proteins mit t-PA-Aktivität erlaubt.

Diese Aufgabe wird erfindungsgemäß dadurch gelöst, daß zur Lösung mindestens zwei Substanzen aus der Gruppe der D- und/oder L-Aminosäuren, deren Salze, Derivate oder Homologe zugesetzt werden.

Es hat sich überraschenderweise gezeigt, daß der Zusatz von mindestens zwei Substanzen aus der Gruppe der D- und/oder L-Aminosäuren, deren Salze, Derivate oder Homologe einen stabilitätsfördernden Einfluß auf Proteine mit t-PA-Aktivität hat. Gleichzeitig kann durch Kombination mehrerer dieser Substanzen eine erhöhte Löslichkeit des Proteins beobachtet werden. Beide Effekte sind in ihrer Ausprägung vollkommen unerwartet. Sowohl die Erhöhung der Stabilität als auch die Steigerung der Löslichkeit beruhen offenbar auf einem Synergismus der Einzelwirkungen.

Die erfindungsgemäßen Substanzen, z.B. Lysin, Ornithin, Arginin, Diaminopimelinsäure, Agmatin, Kreatin, Guanidinoessigsäure, Acetylornithin, Citrullin, Argininobernsteinsäure, Tranexansäure, epsilon-Aminocapronsäure, sind hervorragend zur Formulierung von zur Infusion bestimmten Lösungen geeignet. Ein ihnen allen gemeinsames Merkmal ist die Anwesenheit einer basischen Gruppierung in Form einer Amino- und/oder einer Guanidinogruppe.

Als besonders geeignet zur Herstellung einer Lösung hoher spezifischer Volumenaktivität von einem Protein mit Plasminogenaktivator-Aktivität hat sich eine Kombination von Arginin und Lysin, bevorzugt 0,001 bis 1 mol/l, besonders bevorzugt 0,01 bis 0,5 mol/l, erwiesen. So konnte z.B. gezeigt werden, daß die t-PA-Aktivität in einem Zellkulturüberstand nach 5-tägiger Inkubation bei + 4°C auf 31 % des Ausgangswertes gesunken war, während der Zusatz von 0,1 mol/l Arginin und 0,1 mol/l Lysin zu einer parallelen Probe nach gleicher Inkubationsdauer zu einem Aktivitätsrückgang von nur 5% führte.

Der stabilitätsfördernde und löslichkeitserhöhende Einfluß der erfindungsgemäßen Substanzen konnte sowohl für den Gewebe-Plasminogenaktivator in seiner Ein- oder Zwei-Kettenform als auch für natürlich vorkommende, synthetisch oder gentechnologisch hergestellte Derivate oder auch Kombinationen von t-PA und einem seiner natürlich vorkommenden synthetisch oder gentechnologisch hergestellten Derivate und/oder Pro-Urokinase oder Urokinase beobachtet werden.

Die Isolierung des t-PAs oder eines Proteins mit gleicher Aktivität kann nach üblichen Methoden durchgeführt werden. Gegebenenfalls wird das Protein mit t-PA-Aktivität vorerst durch Dialyse gegen eine gepufferte Lösung eines chaotropen Agenzes, z.B. KSCN, in Lösung gebracht, in diesem Zustand auf die gewünschte t-PA-Konzentration konzentriert, und anschließend gegen eine mindestens zwei der erfindungsgemäßen Substanzen, bevorzugt Arginin und Lysin enthaltende, gepufferte Lösung dialysiert. Beispielsweise wird dabei die Lösung mit einem Protein mit Plasminogenaktivator-Aktivität zuerst gegen etwa 1,6 mol/l KSCN in etwa 0,05 mol/l Tris-HCl pH 7 und anschließend gegen jeweils 0,1 mol/l Arginin und Lysin in 0,05 mol/l Tris-HCl pH 7 dialysiert.

Ebenso aber können die die Stabilität fördernden Effekte durch direkten Zusatz der erfindungsgemäßen Substanzen zum Überstand einer Zellkultur erzielt werden. Die Überstände von erfindungsgemäß behandelten Zellkulturen führen zur Isolierung von Proteinen mit t-PA-Aktivität, deren spezifische Aktivität im Vergleich zu Kontrollseren um ein Dreifaches erhöht ist.

Die stabilitäts- und löslichkeitsfördernden Effekte der erfindungsgemäßen Substanzen sind über einen weiten Bereich pH-unabhängig. Der pH der entsprechenden proteinhaltigen Lösung kann zwischen 5 und 10 liegen und beträgt bevorzugt 6 bis 8.

Üblicherweise werden zur parenteralen Applikation bestimmte Lösungen filtersterilisiert, weil viele der biologisch aktiven Moleküle durch Pasteurisierung zerstört werden würden. Bei filtersterilisierten Produkten kann jedoch die Anwesenheit von Viren nie vollkommen ausgeschlossen werden, wie die SV 40-Kontamination der Pockenschutzimpfung oder die Übertragung des Aids-Virus durch Faktor VIII-Präparate beweisen.

Ein wesentlicher Vorteil des hier beschriebenen Verfahrens besteht darin, daß die Proteine mit t-PA-Aktivität durch den Zusatz erfindungsgemäßer Substanzen so weit stabilisiert werden, daß ihre Aktivität auch nach Pasteurisierung weitgehend erhalten bleibt. Während nach einer Niedrigtemperaturpasteurisierung eine gepufferte t-PA-haltige Lösung z.B. nur noch 0,5 Prozent der Ausgangs-Aktivität aufwies, konnten durch Zusatz von je 1 mol/l Arginin und Lysin ungefähr 85 Prozent der Ausgangs-Aktivität erhalten werden.

Der stabilisierende Effekt der erfindungsgemäßen Substanzen ist von der Art der Pasteurisierung weitgehend unabhängig. So kann die Pasteurisierung bei pH 5 bis 10 durch eine 1- bis 60-stündige Inkubation bei Temperaturen zwischen 40°C undd 90°C durchgeführt werden.

Zweckmäßigerweise wird die Pasteurisierung jedoch bei nahezu physiologischem pH, d.h., zwischen pH 6 und pH 8, durch 6- bis 16-stündige Inkubation bei Temperaturen zwischen 50°C und 70°C durchgeführt.

Die bevorzugteste Ausführungsform ist eine Niedrigtemperaturpasteurisierung bei ungefähr pH 7, d.h., daß das Protein mit t-PA-Aktivität 10 Stunden lang bei 60°C inkubiert wird.

Die proteinhaltige Lösung kann gegebenenfalls mit weiteren Zusätzen, z.B. Mono- oder Disacchariden, Zuckeralkoholen, gegebenenfalls auch anderen Begleitkomponenten, wie beispielsweise Albuminen, Gelatine, Haemaccel, Natriumchlorid, Calciumchlorid, Heparin, ETDA, Glycin oder Detergenzien versetzt werden. Der Zusatz dieser Begleitkomponenten in physiologisch verträglichen Mengen dient z.B. der Stabilisierung, der Pufferung des Systems, der Inhibierung von Proteasen, der Erniedrigung der Oberflächenspannung und der Regulierung der Osmolarität.

Der Zusatz von Saccharose oder Sorbit hat einen weiteren stabilitätsfördernden Effekt bei der Pasteurisierung. So kann der nach einer Pasteurisierung verbleibende Anteil aktiver Moleküle in einer Glycingepufferten t-PA-Lösung, die je 0,5 mol/l Arginin und Lysin enthält, von rund 81 Prozent auf 96 Prozent gesteigert werden. Bevorzugt ist dabei ein Zusatz von 0,2 bis 2 kg Saccharose oder Sorbit pro Liter.

Die Aktivität von t-PA oder Proteinen mit gleicher Wirkung, die gemäß der vorliegenden Erfindung behandelt worden sind, bleibt auch nach Lyophilisation, gefolgt vom Auflösen in sterilisiertem Wasser unmittelbar vor der Anwendung, erhalten.

Durch das erfindungsgemäße Verfahren kann eine Proteinlösung mit t-PA-Aktivität hergestellt werden, deren spezifische Volumenaktivität mehr als $5 \times 10^6$ U/ml beträgt. Diese Konzentration gewährleistet eine problemlose Applikation auch bei kontinuierlicher Infusion über einen längeren Zeitraum. Bei Patienten mit extremen Ausscheidungsschwierigkeiten kann die t-PA-Konzentration bis auf $30 \times 10^6$ U/ml erhöht werden, ohne daß Löslichkeits- oder Stabilitätsprobleme auftreten.

Unabhängig von der gewählten t-PA-Konzentration hat es sich gezeigt, daß die Aktivität erfindungsgemäß hergestellter Lösungen nach der Pasteurisierung im wesentlichen erhalten bleibt. Die erfindungsgemäße wässrige Proteinlösung mit t-PA-Aktivität stellt das erste, durch Pasteurisierung sterilisierte Plasminogenaktivitorpräparat mit einer therapeutisch sinnvollen spezifischen Volumenaktivität dar.

Die erfindungsgemäße Lösung eignet sich für die Verwendung in der Human- und der Veterinärmedizin gleichermaßen. Da ausschließlich physiologisch verträgliche Substanzen zugesetzt werden, treten bei der Applikation der erfindungsgemäßen Lösung weder Entzündungen noch Hautirritationen oder Gefäßschäden auf.

Der bevorzugte Anwendungsbereich der erfindungsgemäßen Lösung sind Therapie und Prophylaxe von Thrombosen und Embolien, d.h., daß das erfindungsgemäße Präparat als Fibrinolytikum verwendet werden kann.

Die Beispiele erläutern die Erfindung.

**Beispiel 1**

t-PA-produzierende CHO-Zellen (Chinese Hamster Ovary) wurden im 20 l" Dulbecco's modified Eaglés Medium", enthaltend 5% Rinderserum, gezüchtet. Die Zellkulturüberstände wurden nach jeweils 24 Std. geerntet und durch frisches Medium ersetzt.

Die steril geernteten Zellkulturüberstände wurden 5 Tage in Anwesenheit bzw. Abwesenheit von 0,1 mol/l Arginin und 0,1 mol/l Lysin bei + 4°C gelagert.

Die t-PA-Aktivität wurde täglich mit der Methode von Ranby (Progress in Fibrinolysis 5, 233-235, 1981) bestimmt.

| Dauer der Inkubation bei 4°C (Tage) | Aktivität in % | |
|---|---|---|
| | Zellkulturüberstand ohne Arg, Lys | Zellkulturüberstand 0,1 mol/l Arg + 0,1 mol/l Lys |
| - | 100 | 100 |
| 1 | 65 | 98 |
| 2 | 52 | 97 |
| 3 | 40 | 96 |
| 4 | 35 | 96 |
| 5 | 31 | 95 |

Die Ergebnisse zeigen eindeutig, daß der Anteil aktiven t-PAs in einem unbehandelten Zellkulturüberstand um fast 70 Prozent zurückgeht, d.h., daß die spezifische Aktivität des anschließend Isolierten Proteins nur knapp 30 Prozent der spezifischen Aktivität zum Zeitpunkt der Ernte beträgt. Im erfindungsgemäß behandelten Zellkulturüberstand jedoch beträgt die Aktivitätsabnahme lediglich 5 Prozent, so daß durch den Zusatz von Arginin und Lysin die vorübergehende Lagerung der Zellkulturüberstände ohne wesentlichen Aktivitätsverlust möglich wird.

**Beispiel 2**

Eine t-PA-haltige Losung wurde gegen 1,6 mol/l KSCN in 0,05 mol/l Tris-HCl pH 7,0 dialysiert, anschließend gegen 0,1 mol/l Arginin und 0,1 mol/l Lysin in 0,05 mol/l Tris-HCl, pH 7,0, dialysiert und auf 0,1, 0,2, 0,5, 1,0, 10, 20, 40, 60 und 80 mg t-PA/ml ankonzentriert. Es wurde beobachtet, daß t-PA in Lösung bis zu einer Konzentration von 60 mg/ml gehalten werden konnte. Mit Tween 80[R] (0,1 %) ohne weitere Zusätze lag die Löslichkeitsgrenze bei o,5 mg t-PA/ml.

**Beispiel 3**

Eine t-PA-enthaltende Lösung wurde gegen 1,6 mol/l KSCN in 0,05 mol/l Tris, pH 7,0, und anschließend gegen einen Puffer, enthaltend 0,05 mol/l Glycin, pH 7,0, sowie steigende Konzentrationen an Arginin und Lysin dialysiert. Wo angegeben, wurde die t-PA-haltige Lösung mit 0,5 bzw. 1 g/ml Saccharose versetzt. Der pH-Wert der Lösungen wurde auf 7 eingestellt. Die Lösungen wurden 10 Std. bei 60°C erhitzt. Die t-PA-Aktivität wurde vor und nach Pasteurisierung bestimmt.

| Konzentration von Arginin + Lysin (mol/l) | | Aktivität nach Pasteurisierung (%) Saccharose-Konzentration (g/ml) | | |
|---|---|---|---|---|
| | | 0 | 0,5 | 1 |
| -- | -- | 0,5 | – | 68 |
| 0,001 | 0,001 | 56,0 | – | – |
| 0,05 | 0,05 | 56,9 | – | – |
| 0,1 | 0,1 | 71,3 | 88,0 | 88,0 |
| 0,2 | 0,2 | 76,1 | – | – |
| 0,5 | 0,5 | 80,7 | 91,0 | 96,3 |
| 1,0 | 1,0 | 85,6 | – | – |

Die Tabelle zeigt, daß sowohl Saccharose als auch der Zusatz von Arginin und Lysin einen stabilisierenden Effekt auf den Plasminogenaktivator haben. Während in einer Probe, der weder Saccharose noch Arginin oder Lysin zugefügt worden sind, die Pasteurisierung die Aktivität fast vollkommen zerstört, bleiben nach Zusatz von einem Gramm Saccharose/ml schon 68 Prozent der Ausgangsaktivitat erhalten. Dieser Wert kann durch Zusatz von Arginin und Lysin erheblich verbessert werden, so daß bei einer Kombination von 1 g/ml Saccharose und je 0,5 mol/l Arginin und Lysin 96 Prozent der Ausgangs-Aktivität erhalten bleiben, wodurch der Aktivitätsverlust durch Pasteurisierung vernachlässigbar klein wird.

**Patentansprüche**
**Patentansprüche für folgende Vertragsstaaten : AT, BE, CH, DE, FR, GB, IT, LI, LU, NL, SE**

1. Verfahren zur Herstellung einer Lösung von einem Protein mit Gewebeplasminogen-Aktivator (t-PA)-Aktivität fit einer spezifischen Volumenaktivität größer als $5 \times 10^6$ U/ml, dadurch gekennzeichnet, daß zum Lösen mindestens zwei Substanzen aus der Gruppe der D-und/oder L-Aminosäuren enthaltend basische Funktionen in Form einer Amino- und/oder Guanidinogruppe, ausgewählt aus der Gruppe Lysin, Ornithin, Arginin, Diaminopimelinsäure, Agmatin, Kreatin, Guanidinoessigsäure, Acetylornithin, Citrullin, Argininobersteinsäure, Tranexansäure und $\Sigma$-Aminocapronsäure, ihrer Salze oder Derivate zugesetzt werden.

2. Verfahren nach Anspruch 1, dadurch gekennzeichnet, daß Arginin und Lysin, bevorzugt jeweils 0,001 bis 1 mol/l, besonders bevorzugt 0,01 bis 0,5 mol/l, zugesetzt werden.

3. Verfahren nach mindestens einem der Ansprüche 1 oder 2, dadurch gekennzeichnet, daß das Protein Gewebe-Plasminogenaktivator in der Ein- oder Zweikettenform oder eines seiner natürlich vorkommenden, synthetisch oder gentechnologisch hergestellten Derivate ist und alleine oder in Kombination aus t-PA und einem seiner naturlich vorkommenden synthetisch oder gentechnologisch hergestellten Derivate und/oder Pro-Urokinase oder Urokinase in der Lösung enthalten ist.

4. Verfahren nach mindestens einem der Ansprüche 1 bis 3, dadurch gekennzeichnet, daß eine Lösung mit einem Protein mit Plasminogenaktivator-Aktivität zuerst gegen eine gepufferte KSCN-Lösung, anschließend gegen eine gepufferte Lösung, enthaltend Arginin und Lysin, dialysiert wird, vorzugsweise zuerst gegen etwa 1,6 mol/l KSCN in etwa 0,05 mol/l Tris-HCl pH 7, anschliessend gegen 0,1 mol/l Arginin, 0,1 mol/l Lysin pH 7.

5. Verfahren nach mindestens einem der Ansprüche 1 bis 3, dadurch gekennzeichnet, daß die Lösung der Überstand einer Zellkultur ist.

6. Verfahren nach mindestens einem der Ansprüche 1 bis 5, dadurch gekennzeichnet, daß die Lösung auf pH 5 bis 10, bevorzugt pH 6 bis 8 eingestellt wird.

7. Verfahren nach mindestens einem der Ansprüche 1 bis 6, dadurch gekennzeichnet, daß die proteinhaltige Lösung pasteurisiert wird.

8. Verfahren nach Anspruch 7, dadurch gekennzeichnet, daß die Pasteurisierung bei pH 5 bis 10 durch eine ein- bis 60-stündige Inkubation bei 40°C bis 90°C durchgeführt wird.

9. Verfahren nach Anspruch 8, dadurch gekennzeichnet, daß die Pasteurisierung bei pH 6 bis 8 durch 6- bis 15-stündige Inkubation bei 50°C bis 70°C durchgeführt wird.

10. Verfahren nach Anspruch 9, dadurch gekennzeichnet, daß die Pasteurisierung bei ph 6,5 bis 7,5 durch 10 Stunden Inkubation bei 60°C durchgeführt wird.

11. Verfahren nach mindestens einem der Ansprüche 1 bis 10, dadurch gekennzeichnet, daß der Lösung zusätzlich übliche, pharmazeutisch verträgliche, stabilisierende und/oder puffernde Substanzen zugefügt werden.

12. Verfahren nach Anspruch 11, dadurch gekennzeichnet, daß Saccharose und/oder Sorbit, bevorzugt 0,2 bis 2 kg/l, zugesetzt werden.

13. Verfahren nach mindestens einem der Ansprüche 1 bis 12, dadurch gekennzeichnet, daß die proteinhaltige Lösung lyophilisiert wird.

14. Lösung erhalten nach dem Verfahren gemäß mindestens einem der Ansprüche 1 bis 13, dadurch gekennzeichnet, daß die Aktivität nach der Pasteurisierung im wesentlichen erhalten bleibt.

15. Lösung erhalten nach mindestens einem der Ansprüche 1 bis 13 zur Verwendung in der Human- oder Veterinärmedizin.

16. Lösung nach Anspruch 15 zur Verwendung als Fibrinolytikum.

**Patentansprüche für folgende Vertragsstaaten : ES, GR**

1. Verfahren zur Herstellung und gegebenenfalls Pasteurisierung einer Lösung von einem Protein mit Gewebeplasminogen-Aktivator (t-PA)-Aktivität mit einer spezifischen Volumenaktivität größer als $5 \times 10^6$ U/ml, dadurch gekennzeichnet, daß zum Lösen mindestens zwei Substanzen aus der Gruppe der D- und/oder L-Aminosäuren enthaltend basische Funktionen in Form einer Amino- und/oder Guanidinogruppe, ausgewählt aus der Gruppe Lysin, Ornithin, Arginin, Diaminopimelinsäure, Agmatin, Kreatin, Guanidinoessigsäure, Acetylornithin, Citrullin, Argininobersteinsäure, Tranexansäure und $\Sigma$-Aminoca-pronsäure, ihrer Salze oder Derivate zugesetzt werden.

2. Verfahren nach Anspruch 1, dadurch gekennzeichnet, daß Arginin und Lysin, bevorzugt jeweils 0,001 bis 1 mol/l, besonders bevorzugt 0,01 bis 0,5 mol/l, zugesetzt werden.

3. Verfahren nach mindestens einem der Ansprüche 1 oder 2, dadurch gekennzeichnet, daß das Protein Gewebe-Plasminogenaktivator in der Ein- oder Zweikettenform oder eines seiner natürlich vorkommenden, synthetisch oder gentechnologisch hergestellten Derivate ist und alleine oder in Kombination aus t-PA und einem seiner natürlich vorkommenden synthetisch oder gentechnologisch hergestellten Derivate und/oder Pro-Urokinase oder Urokinase in der Lösung enthalten ist.

4. Verfahren nach mindestens einem der Ansprüche 1 bis 3, dadurch gekennzeichnet, daß eine Lösung mit einem Protein mit Plasminogenaktivator-Aktivität zuerst gegen eine gepufferte KSCN-Lösung, anschließend gegen eine gepufferte Lösung, enthaltend Arginin und Lysin, dialysiert wird, vorzugsweise zuerst gegen etwa 1,6 mol/l KSCN in etwa 0,05 mol/l Tris-HCl pH 7, anschliessend gegen 0,1 mol/l Arginin, 0,1 mol/l Lysin pH 7.

5. Verfahren nach mindestens einem der Ansprüche 1 bis 3, dadurch gekennzeichnet, daß die Lösung der Überstand einer Zellkultur ist.

6. Verfahren nach mindestens einem der Ansprüche 1 bis 5, dadurch gekennzeichnet, daß die Lösung auf pH 5 bis 10, bevorzugt pH 6 bis 8 eingestellt wird.

7. Verfahren nach mindestens einem der Ansprüche 1 bis 6, dadurch gekennzeichnet, daß die proteinhaltige Lösung pasteurisiert wird.

8. Verfahren nach Anspruch 7, dadurch gekennzeichnet, daß die Pasteurisierung bei pH 5 bis 10 durch eine ein- bis 60-stündige Inkubation bei 40°C bis 90°C durchgeführt wird.

9. Verfahren nach Anspruch 8, dadurch gekennzeichnet, daß die Pasteurisierung bei pH 6 bis 8 durch 6- bis 15-stündige Inkubation bei 50°C bis 70°C durchgeführt wird.

10. Verfahren nach Anspruch 9, dadurch gekennzeichnet, daß die Pasteurisierung bei ph 6,5 bis 7,5 durch 10 Stunden Inkubation bei 60°C durchgeführt wird.

11. Verfahren nach mindestens einem der Ansprüche 1 bis 10, dadurch gekennzeichnet, daß der Lösung zusätzlich übliche, pharmazeutisch verträgliche, stabilisierende und/oder puffernde Substanzen zugefügt werden.

12. Verfahren nach Anspruch 11, dadurch gekennzeichnet, daß Saccharose und/oder Sorbit, bevorzugt 0,2 bis 2 kg/l, zugesetzt werden.

13. Verfahren nach mindestens einem der Ansprüche 1 bis 12, dadurch gekennzeichnet, daß die proteinhaltige Lösung lyophilisiert wird.

**Claims**
**Claims for the following Contracting States : AT, BE, CH, DE, FR, GB, IT, LI, LU, NL, SE**

1. A process for the preparation of a solution of a protein having tissue plasminogen activator (t-PA) activity having a specific activity concentration of more than $5 \times 10^6$ U/ml, which comprises adding to dissolve at least two substances from the group of D- and/or L-amino acids containing basic groups in the form of an amino and/or guanidino group, selected from the group comprising lysine, ornithine, arginine, diaminopimelic acid, agmatine, creatine, guanidinoacetic acid, acetylornithine, citrulline, argininosuccinic acid, tranexamic acid and $\epsilon$-aminocaproic acid, their salts or derivatives.

2. The process as claimed in claim 1, wherein arginine and lysine are added, preferably 0.001 to 1 mol/l of each, particularly preferably 0.01 to 0.5 mol/l.

3. The process as claimed in at least one of claims 1 or 2, wherein the protein tissue plasminogen activator is in the one- or two-chain form or is one of its derivatives which occurs naturally or has been prepared by synthesis or genetic manipulation, and is contained in the solution alone or in a combination of t-PA and of one of its derivatives which occurs naturally or has been prepared by synthesis or genetic manipulation, and/or prourokinase or urokinase.

4. The process as claimed in at least one of claims 1 to 3, wherein a solution containing a protein having plasminogen activator activity is dialyzed first against a buffered KSCN solution and then against a buffered solution containing arginine and lysine, preferably first against about 1.6 mol/l KSCN in about 0.05 mol/l tris-HCl, pH 7, and then against 0.1 mol/l arginine, 0.1 mol/l lysine, pH 7.

5. The process as claimed in at least one of claims 1 to 3, wherein the solution is the supernatant of a cell culture.

6. The process as claimed in at least one of claims 1 to 5, wherein the solution is adjusted to pH 5 to 10, preferably pH 6 to 8.

7. The process as claimed in at least one of claims 1 to 6, wherein the protein-containing solution is pasteurized.

8. The process as claimed in claim 7, wherein the pasteurization is carried out at pH 5 to 10 by incubation as 40°C to 90°C for one to 60 hours.

9. The process as claimed in claim 8, wherein the pasteurization is carried out at pH 6 to 8 by incubation at 50°C to 70°C for 6 to 15 hours.

10. The process as claimed in claim 9, wherein the pasteurization is carried out at pH 6.5 to 7.5 by incubation at 60°C for 10 hours.

11. The process as claimed in at least one of claims 1 to 10, wherein customary pharmaceutically tolerated stabilizing and/or buffering substances are added to the solution in addition.

12. The process as claimed in claim 11, wherein sucrose and/or sorbitol, preferably 0.2 to 2 kg/l, are added.

13. The process as claimed in at least one of claims 1 to 12, wherein the protein-containing solution is freeze-dried.

14. A solution obtained by the process as claimed in at least one of claims 1 to 13, wherein the activity is essentially retained after pasteurization.

15. A solution obtained as claimed in at least one of claims 1 to 13 for use in human or veterinary medicine.

16. A solution as claimed in claim 15 for use as fibrinolytic.

**Claims for the following Contracting States : ES, GR**

1. A process for the preparation and, if appropriate, pasteurization of a solution of a protein having tissue plasminogen activator (t-PA) activity having a specific activity concentration of more than $5 \times 10^6$ U/ml, which comprises adding to dissolve at least two substances from the group of D- and/or L-amino acids containing basic groups in the form of an amino and/or guanidino group, selected from the group comprising lysine, ornithine, arginine, diaminopimelic acid, agmatine, creatine, guanidinoacetic acid, acetylornithine, citrulline, argininosuccinic acid, tranexamic acid and $\epsilon$-aminocaproic acid, their salts or derivatives.

2. The process as claimed in claim 1, wherein arginine and lysine are added, preferably 0.001 to 1 mol/l of each, particularly preferably 0.01 to 0.5 mol/l.

3. The process as claimed in at least one of claims 1 or 2, wherein the protein tissue plasminogen activator is in the one- or two-chain form or is one of its derivatives which occurs naturally or has been prepared by synthesis or genetic manipulation, and is contained in the solution alone or in a combination of t-PA and of one of its derivatives which occurs naturally or has been prepared by synthesis or genetic manipulation, and/or prourokinase or urokinase.

4. The process as claimed in at least one of claims 1 to 3, wherein a solution containing a protein having plasminogen activator activity is dialyzed first against a buffered KSCN solution and then against a buffered solution containing arginine and lysine, preferably first against about 1.6 mol/l KSCN in about 0.05 mol/l tris-HCl, pH 7, and then against 0.1 mol/l arginine, 0.1 mol/l lysine, pH 7.

5. The process as claimed in at least one of claims 1 to 3, wherein the solution is the supernatant of a cell culture.

6. The process as claimed in at least one of claims 1 to 5, wherein the solution is adjusted to pH 5 to 10, preferably pH 6 to 8.

7. The process as claimed in at least one of claims 1 to 6, wherein the protein-containing solution is pasteurized.

8. The process as claimed in claim 7, wherein the pasteurization is carried out at pH 5 to 10 by incubation at 40°C to 90°C for one to 60 hours.

9. The process as claimed in claim 8, wherein the pasteurization is carried out at pH 6 to 8 by incubation at 50°C to 70°C for 6 to 15 hours.

10. The process as claimed in claim 9, wherein the pasteurization is carried out at pH 6.5 to 7.5 by incubation at 60°C for 10 hours.

11. The process as claimed in at least one of claims 1 to 10, wherein customary pharmaceutically tolerated stabilizing and/or buffering substances are added to the solution in addition.

12. The process as claimed in claim 11, wherein sucrose and/or sorbitol, preferably 0.2 to 2 kg/l, are added.

13. The process as claimed in at least one of claims 1 to 12, wherein the protein-containing solution is freeze-dried.

**Revendications**
**Revendications pour les Etats contractants suivants : AT, BE, CH, DE, FR, GB, IT, LI, LU, NL, SE**

1. Procédé pour la préparation d'une solution d'une protéine à activité d'activateur tissulaire du plasminogène (tPA) ayant une activité volumique spécifique de plus de $5 \times 10^6$ U/ml, caractérisé en ce que, pour la dissolution, on ajoute au moins deux substances appartenant au groupe des D- et/ou L-aminoacides contenant des fonctions basiques sous forme d'un groupe amino et/ou d'un groupe guanidino, choisies parmi la lysine, l'ornithine, l'arginine, l'acide diaminopimélique, l'Agmatine, la créatine, l'acide guanidinoacétique, l'acétylornithine, la citrulline, l'acide argininosuccinique, l'acide tranexamique et l'acide $\epsilon$-aminoproïque, leurs sels ou dérivés.

2. Procédé selon la revendication 1, caractérisé en ce que l'on ajoute de l'arginine et de la lysine, de préférence à raison de 0,001 à 1 mole/l chaque, en particulier de 0,01 à 0,5 mole/l chaque.

**3.** Procédé selon au moins l'une des revendications 1 ou 2, caractérisé en ce que la protéine activateur tissulaire du plasminogène est sous forme mono- ou bicaténaire ou d'un de ses dérivés existant dans la nature, synthétiques ou produits par génie génétique, et est contenue seule ou en association de tPA et d'un de ses dérivés existant dans la nature, synthétiques ou produits par génie génétique, et/ou de pro-urokinase ou d'urokinase.

**4.** Procédé selon au moins l'une des revendications 1 à 3, caractérisé en ce qu'une solution contenant une protéine à activité d'activateur du plasminogène est dialysée tout d'abord contre une solution de KSCN tamponnée, ensuite contre une solution tamponnée contenant de l'arginine et de la lysine, de préférence tout d'abord contre environ 1,6 mole/l de KSCN dans du tampon Tris-HCl à environ 0,05 mole/l, pH 7, puis contre 0,1 mole/l d'arginine, 0,1 mole/l de lysine, pH 7.

**5.** Procédé selon au moins l'une des revendications 1 à 3, caractérisé en ce que la solution est le surnageant d'une culture de cellules.

**6.** Procédé selon au moins l'une des revendications 1 à 5, caractérisé en ce que la solution est ajustée à pH 5 à 10, de préférence à pH 6 à 8.

**7.** Procédé selon au moins l'une des revendications 1 à 6, caractérisé en ce que la solution contenant une protéine est pasteurisée.

**8.** Procédé selon la revendication 7, caractérisé en ce que l'on effectue la pasteurisation à pH 5-10, au moyen d'une incubation à 40-90°C pendant 1 à 60 heures.

**9.** Procédé selon la revendication 8, caractérisé en ce que l'on effectue la pasteurisation à pH 6-8 par incubation à 50-70°C pendant 6 à 15 heures.

**10.** Procédé selon la revendication 9, caractérisé en ce que l'on effectue la pasteurisation à pH 6,5-7,5 par incubation à 60°C pendant 10 heures.

**11.** Procédé selon au moins l'une des revendications 1 à 10, caractérisé en ce que l'on ajoute en outre à la solution des substances usuelles stabilisatrices et/ou tampon, pharmaceutiquement acceptables.

**12.** Procédé selon la revendication 11, caractérisé en ce que l'on ajoute du saccharose et/ou du sorbitol, de préférence à raison de 0,2 à 2 kg/l.

**13.** Procédé selon au moins l'une des revendications 1 à 12, caractérisé en ce que la solution contenant une protéine est lyophilisée.

**14.** Solution obtenue conformément au procédé selon au moins l'une des revendications 1 à 13, caractérisée en ce que l'activité est pratiquement maintenue après la pasteurisation.

**15.** Solution obtenue selon au moins l'une des revendications 1 à 13, pour utilisation en médecine humaine ou vétérinaire.

**16.** Solution selon la revendication 15, pour utilisation en tant qu'agent fibrinolytique.

**Revendications pour les Etats contractants suivants : ES, GR**

**1.** Procédé pour la préparation et éventuellement la pasteurisation d'une solution d'une protéine à activité d'activateur tissulaire du plasminogène (tPA) ayant une activité volumique spécifique de plus de 5 x $10^6$ U/ml, caractérisé en ce que, pour la dissolution, on ajoute au moins deux substances appartenant au groupe des D- et/ou L-aminoacides contenant des fonctions basiques sous forme d'un groupe amino et/ou d'un groupe guanidino, choisies parmi la lysine, l'ornithine, l'arginine, l'acide diaminopimélique, l'Agmatine, la créatine, l'acide guanidinoacétique, l'acétylornithine, la citrulline, l'acide argininosuccinique, l'acide tranexamique et l'acide $\epsilon$-aminocaproïque, leurs sels ou dérivés.

**2.** Procédé selon la revendication 1, caractérisé en ce que l'on ajoute de l'arginine et de la lysine, de préférence à raison de 0,001 à 1 mole/l chaque, en particulier de 0,01 à 0,5 mole/l chaque.

3. Procédé selon au moins l'une des revendications 1 ou 2, caractérisé en ce que la protéine activateur tissulaire du plasminogène est sous forme mono- ou bicaténaire ou d'un de ses dérivés existant dans la nature, synthétiques ou produits par génie génétique, et est contenue seule ou en association de tPA et d'un de ses dérivés existant dans la nature, synthétiques ou produits par génie génétique, et/ou de pro-urokinase ou d'urokinase.

4. Procédé selon au moins l'une des revendications 1 à 3, caractérisé en ce qu'une solution contenant une protéine à activité d'activateur du plasminogène est dialysée tout d'abord contre une solution de KSCN tamponnée, ensuite contre une solution tamponnée contenant de l'arginine et de la lysine, de préférence tout d'abord contre environ 1,6 mole/l de KSCN dans du tampon Tris-HCl à 0,05 mole/l, pH 7, puis contre 0,1 mole/l d'arginine, 0,1 mole/l de lysine, pH 7.

5. Procédé selon au moins l'une des revendications 1 à 3, caractérisé en ce que la solution est le surnageant d'une culture de cellules.

6. Procédé selon au moins l'une des revendications 1 à 5, caractérisé en ce que la solution est ajustée à pH 5 à 10, de préférence à pH 6 à 8.

7. Procédé selon au moins l'une des revendications 1 à 6, caractérisé en ce que la solution contenant une protéine est pasteurisée.

8. Procédé selon la revendication 7, caractérisé en ce que l'on effectue la pasteurisation à pH 5-10, au moyen d'une incubation à 40-90°C pendant 1 à 60 heures.

9. Procédé selon la revendication 8, caractérisé en ce que l'on effectue la pasteurisation à pH 6-8 par incubation à 50-70°C pendant 6 à 15 heures.

10. Procédé selon la revendication 9, caractérisé en ce que l'on effectue la pasteurisation à pH 6,5-7,5 par incubation à 60°C pendant 10 heures.

11. Procédé selon au moins l'une des revendications 1 à 10, caractérisé en ce que l'on ajoute en outre à la solution des substances usuelles stabilisatrices et/ou tampon, pharmaceutiquement acceptables.

12. Procédé selon la revendication 11, caractérisé en ce que l'on ajoute du saccharose et/ou du sorbitol, de préférence à raison de 0,2 à 2 kg/l.

13. Procédé selon au moins l'une des revendications 1 à 12, caractérisé en ce que la solution contenant une protéine est lyophilisée.